# EUROPÄISCHE PATENTSCHRIFT

(11) **EP 2 635 218 B1**
(45) Veröffentlichungstag und Bekanntmachung des Hinweises auf die Patenterteilung: **09.01.2019**
(21) Anmeldenummer: 11781504.3
(22) Anmeldetag: 04.11.2011
(51) Int. Cl.: A61B 18/14, A61B 18/00, A61B 18/04

(54) **ELEKTRODENEINRICHTUNG EINES ELEKTROCHIRURGISCHEN INSTRUMENTS**
ELECTRODE ARRANGEMENT OF AN ELECTROSURGICAL INSTRUMENT
DISPOSITIF À ÉLECTRODES D'UN INSTRUMENT D'ÉLECTROCHIRURGIE

(30) Priorität: 04.11.2010 DE 102010060336
(43) Veröffentlichungstag der Anmeldung: 11.09.2013
(73) Patentinhaber: Erbe Elektromedizin GmbH, 72072 Tübingen (DE)
(72) Erfinder: KÜHNER, Ralf, 70567 Stuttgart-Möhringen (DE); KARWEI, Dietmar, 72131 Ofterdingen (DE)
(74) Vertreter: Rüger Abel
(86) Internationale Anmeldenummer: PCT/EP2011/069435
(87) Internationale Veröffentlichungsnummer: WO 2012/059587

(56) Entgegenhaltungen:
- WO-A1-99/40858
- DE-A1- 19 652 098
- US-A- 5 665 085

## Beschreibung

Die Erfindung betrifft eine Elektrodeneinrichtung eines elektrochirurgischen Instruments.

In der Chirurgie werden vielfach Instrumente genutzt, die mit Hilfe von hochfrequenten Strömen Gewebe schneiden und koagulieren können. Derartige Instrumente werden vielfach aus Edelstahl hergestellt, jedoch kann es bei solchen Instrumenten zu einem Anbacken des Gewebes bzw. der entstehenden Stoffe am Instrument kommen. Ein Reinigen der Instrumente ist aufwändig.

Um das Anbacken zu vermeiden oder zumindest zu verringern wird In der US 5,925,039 vorgeschlagen, einen metallischen Trägerkörper mit einer leitenden Keramik zu beschichten. Derartige Instrumente sind jedoch nur relativ wenig haltbar, weil die sehr unterschiedlichen Ausdehnungskoeffizienten von Keramik und Metallträger beim Erwärmen der Instrumente zu Abplatzungen führen.

Aus der US 4,862,890 ist eine Elektrodeneinrichtung bekannt, bei welcher auf einem keramischen Träger eine Metallbeschichtung aufgebracht wird. Aufgrund der Elastizität der Metallbeschichtung sind die vorgenannten Spannungsprobleme beim Erwärmen der Elektrode zwar nicht so deutlich, jedoch kommt es hier wieder relativ leicht zu einem Anbacken.

Aus der US 5,665,085 ist eine Elektrodeneinrichtung bekannt, bei welcher eine isolierende Keramik mit einer leitenden Keramikbeschichtung vorgeschlagen wird. Die Herstellung dieser Elektrodeneinrichtung bzw. des damit versehenen elektrochirurgischen Instrumentes ist aufwändig und die Haltbarkeit ist nicht hinreichend.

Aus der DE 196 52 098 A1 sind ein chirurgisches Instrument und ein Verfahren zu seiner Herstellung bekannt.

Der Erfindung liegt die Aufgabe zu Grunde, eine Elektrodeneinrichtung der zuvor genannten Art dahin gehend weiterzubilden, dass bei einer vereinfachten Herstellung eine erhöhte Haltbarkeit erzielbar ist.

Diese Aufgabe wird durch eine Elektrodeneinrichtung nach Anspruch 1 und ein Verfahren zu seiner Herstellung nach Anspruch 7 gelöst.
Insbesondere wird diese Aufgabe durch eine Elektrodeneinrichtung eines elektrochirurgischen Instruments, umfassend mindestens einen leitfähigen Elektrodenabschnitt und einen elektrisch isolierenden Trägerabschnitt, wobei der Elektrodenabschnitt ebenso wie der Trägerabschnitt aus einem keramischen Material gefertigt sind, dadurch gelöst, dass ein Grünling des Trägerabschnittes zusammen mit einem Grünling des Elektrodenabschnittes insbesondere stofflich zu einem einzigen Verbundgrünling verbunden und gemeinsam gesintert sind.
Ein wesentlicher Punkt der Erfindung besteht also darin, dass die beiden Keramikstoffe schon als Grünlinge zusammengefügt und damit an den Grenzflächen ineinander übergehend ausgebildet sind, bevor sie gemeinsam gesintert werden. Diese Herstellungsweise ist in einem Schnittbild sehr gut zu sehen bzw. sehr gut von den bisher üblichen Elektrodeneinrichtungen zu unterscheiden.
Der Elektrodenabschnitt und der Trägerabschnitt sind vorzugsweise aus Keramikmaterial mit im Wesentlichen denselben Wärmeausdehnungskoeffizienten gefertigt. Dadurch entsteht ein Temperaturschwankungen gegenüber unempfindlicher Körper. Vorzugsweise ist das keramische Material in beiden Fällen Siliziumnitrid, jedoch mit unterschiedlichen Dotierungen oder Beimischungen Leitfähigkeit erzeugender Bestandteile, wie diese allgemein bekannt sind. Insbesondere können diese Beimischungen Aluminiumoxid, Yttriumoxid oder Magnesiumoxid umfassen.
Der Verbundgrünling wird vorzugsweise vor dem Sintern einer Oberflächenbehandlung, insbesondere mittels Lasern oder spanabhebende Verformung, z.B. Gleitschleifen unterzogen, was aufgrund der Weichheit des Grünlings zu hervorragenden Oberflächenstrukturen führt.

Bei einer bevorzugten Ausführungsform der Erfindung ist der Elektrodenabschnitt mit dem Trägerabschnitt unter Bildung von Hinterschneidungen verbunden, was die Stabilität der Anordnung erhöht und auch das Herstellen des Verbundgrünlings vereinfacht und den Verbundgrünling stabilisiert.

Der Elektrodenabschnitt kann aufgrund der Leitfähigkeit der verwendeten Keramik ohne weitere Behandlung verwendet werden. Bei alternativen Ausführungsformen der Erfindung wird jedoch der Elektrodenabschnitt mindestens teilweise beschichtet, insbesondere metallisiert bzw. galvanisiert. Dadurch können z.B. diejenigen Abschnitte des Elektrodenabschnitts, welche mit Zuleitungsdrähten elektrisch verbunden werden, mit einem besonders niedrigen Übergangswiderstand ausgestattet werden.

Weiterhin wird die oben genannte Aufgabe durch ein Verfahren zur Herstellung einer Elektrodeneinrichtung eines elektrochirurgischen Instruments durch die folgenden Schritte gelöst:
a) Zurverfügungstellen eines elektrisch isolierenden ersten Keramikmaterials;
b) Zurverfügungstellung eines elektrisch leitenden zweiten Keramikmaterials;
c) Herstellen eines Verbundgrünlings, der mindestens einen Trägerabschnitt aus dem ersten Keramikmaterial und mindestens einen Elektrodenabschnitt aus dem zweiten Keramikmaterial umfasst;
d) gemeinsames Sintern des ersten und des zweiten Keramikmaterials, die zusammen den Verbundgrünling bilden.

Durch das Verbinden der beiden Grünlinge zu einem einzigen Verbundgrünling und das anschließende Sintern wird eine besonders stabile Struktur in einfacher Weise erreicht.

Der Grünling wird vor dem Sintern vorzugsweise einer Oberflächenbehandlung insbesondere durch Lasern oder einer spanabhebenden Verformung, z.B. Gleitschleifen unterworfen. Dadurch können in einfacher Weise hohe Oberflächenqualitäten und auch sehr feine Strukturen erzielt werden.

Vorzugsweise weisen das erste und das zweite Keramikmaterial im Wesentlichen dieselben Wärmeausdehnungskoeffizienten auf, insbesondere umfassen sie dasselbe keramische Material und zwar insbesondere Siliziumnitrid, wobei aber unterschiedliche Dotierungen oder Beimischungen von eine Leitfähigkeit erzeugenden Bestandteilen vorgesehen sind. Dadurch können sehr stabile und temperaturunempfindliche Instrumente hergestellt werden.

Besonders einfach und zuverlässig funktioniert die Herstellung dann, wenn der Verbundgrünling in einem 2K-Spritzgussverfahren (Zweikomponenten-Spritzgussverfahren) in an sich bekannter Herstellungsweise hergestellt wird.

An dieser Stelle sei betont, dass eine Vielzahl von Keramiken Verwendung finden kann, wenn es nur gewährleistet ist, dass die leitende wie auch die nichtleitende Keramik zumindest sehr ähnliche Wärmeausdehnungskoeffizienten aufweisen.

Eine besonders vorteilhafte Verwendung einer derart ausgebildeten bzw. hergestellten Elektrodeneinrichtung besteht darin, sie als Teil eines elektrochirurgischen Schneid- und/oder Koagulationswerkzeugs zu verwenden, bei welchem der Elektrodenabschnitt direkt mit zu behandelndem Gewebe in Kontakt gelangt. Ein Anbacken ist bei einer derartigen Elektrodeneinrichtung kaum zu beobachten.

Eine weitere vorteilhafte Verwendung besteht darin, ein Präparationsinstrument aufzubauen, wobei der Trägerabschnitt einen insbesondere haken-, kugel-, halbkugel- oder scheibenförmigen Präparationsabschnitt und der Elektrodenabschnitt einen Schneid- oder Koagulationsabschnitt des Präparationsinstrumentes bilden.

Eine weitere vorteilhafte Verwendung besteht darin, die Elektrodeneinrichtung in einem Wasserstrahlchirurgieinstrument zu verwenden, wobei der Trägerabschnitt ein Lumen zum Durchleiten eines Schneidfluids umfasst und die Elektrodeneinrichtung zum elektrochirurgischen Schneiden oder zum Koagulieren dient.

Alternativ oder aber auch zusätzlich ist eine Verwendung in einem Plasmachirurgieinstrument möglich, wobei der Elektrodenabschnitt eine vorzugsweise durchströmbare Ionisationselektrode bildet.

Insgesamt lassen sich alle vorgenannten Instrumente bzw. Elektrodeneinrichtungen in vorteilhafter Weise in endoskopischen Chirurgieinstrumenten verwenden, da eine Miniaturisierung sehr gut möglich ist.

Nachfolgend wird die Erfindung anhand von Abbildungen näher erläutert. Hierbei zeigen
- Fig. 1: ein spatelförmiges, elektrochirurgisches Instrument in einem Teilschnitt,
- Fig. 2: das Instrument nach Fig. 1 in ungeschnittenem Zustand,
- Fig. 3: den Spatel-Abschnitt des Instrumentes nach den Fig. 1 und 2 und zwar in einem ersten Herstellungsschritt,
- Fig. 4: einen Schnitt entlang der Linie A-A aus Fig. 3,
- Fig. 5: eine schematisierte Darstellung des Instrumentes nach den Fig. 1 bis 4 in einem zweiten Herstellungsschritt,
- Fig. 6: einen Schnitt entlang der Linie B-B aus Fig. 5,
- Fig. 7: einen Endabschnitt eines weiteren elektrochirurgischen Instruments in einem Längsschnitt,
- Fig. 8: eine weitere Ausführungsform der Erfindung eines elektrochirurgischen Instrumentes in einem Längsschnitt,
- Fig. 9: das Instrument aus Fig. 8 in Draufsicht und
- Fig. 10: den Endabschnitt einer Fasszange gemäß einer Ausführungsform der Erfindung in einem Längsschnitt.

In der nachfolgenden Beschreibung werden für gleiche und gleich wirkende Teile dieselben Bezugsziffern verwendet.

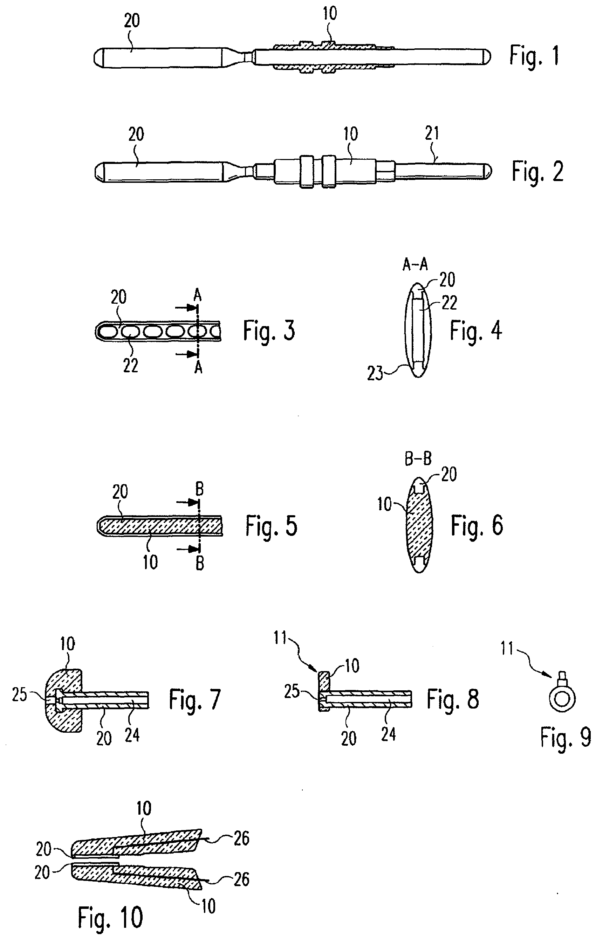

In den Fig. 1 und 2 ist ein spatelförmiges elektrochirurgisches Instrument gezeigt, wie dies allgemein Anwendung findet. Hier ist auf einen Elektrodenabschnitt 20 ein Trägerabschnitt 10 aufgebracht. Der Trägerabschnitt 10 ist isolierend, während der Elektrodenabschnitt 20 elektrisch leitend ist. In beiden Fällen findet eine Siliziumnitrid-Keramik Anwendung, wobei das Siliziumnitrid des elektrisch leitenden Elektrodenabschnitts 20 eine Dotierung mit Aluminiumoxid, Yttriumoxid und Magnesiumoxid aufweist. Diese Dotierung führt dazu, dass zwar der Ausdehnungskoeffizient gegenüber undotiertem Siliziumnitrid praktisch gleich bleibt, die elektrische Leitfähigkeit ist jedoch zumindest so gut, dass eine Verwendung als Elektrode ohne Weiteres möglich ist.

Der dem nichtleitenden Trägerabschnitt 10 nachgelagerte Steckerabschnitt, über welchen das Instrument in einen üblichen Handgriff eingesetzt wird, weist eine Beschichtung 21 aus Metall auf, um einen möglichst geringen Übergangswiderstand zu den Anschlusselementen sicherzustellen.

Nachfolgend wird die Herstellung des spateiförmigen elektrochirurgischen Instruments nach den Fig. 1 und 2 erläutert.

Zunächst wird ein Grünling aus elektrisch leitendem Siliziumnitrid-Material hergestellt, der die in den Fig. 3 und 4 gezeigte Form aufweist. In diesem Grünling sind Öffnungen 22 sowie Hinterschneidungen 23 vorgesehen. In einem nächsten Schritt wird in einem an sich bekannten 2K-Verfahren nichtleitendes Siliziumnitrid an den Grünling nach den Fig. 3 und 4 angespritzt, so dass die Öffnungen 22 und die Hinterschneidungen 23 ausgefüllt werden. Der so gebildete "Verbundgrünling" besteht also aus teilweise dotiertem und teilweise undotiertem Siliziumnitrid, weist also Elektrodenabschnitte 20 und Trägerabschnitte 10 auf. Anschließend an diesen Spritzprozess wird der Verbundgrünling in an sich bekannter Weise gebrannt bzw. gesintert, so dass ein äußerst stabiler Gegenstand - wie in den Fig. 1 und 2 gezeigt - entstanden ist. Die Stabilität ist insbesondere deshalb so hoch, weil die Grenzflächen zwischen dem leitenden und dem nichtleitenden Siliziumnitrid "verschwimmen" und aufgrund der geringen Dotierungsmengen in einem Schliff- oder Schnittbild praktisch nicht mehr zu erkennen sind. Dadurch ist es auch möglich, ein erfindungsgemäßes elektrochirurgisches Instrument von einem Instrument zu unterscheiden, bei welchem auf einem nichtleitenden Siliziumnitrid-Keramik-Körper eine elektrisch leitende Keramik aufgebracht wurde.

An dieser Stelle sei nochmals darauf hingewiesen, dass Siliziumnitrid natürlich nicht die einzige Keramik ist, die zur Herstellung erfindungsgemäßer Instrumente bzw. zur Durchführung des erfindungsgemäßen Verfahrens geeignet ist. Die Literatur gibt hier eine Vielzahl von weiteren Beispielen.

Bei den in den Fig. 7, 8 und 9 gezeigten Ausführungsformen der Erfindung sind Endabschnitte eines elektrochirurgischen Instrumentes gezeigt. Diese weisen jeweils einen rohrförmigen Körper, der aus elektrisch leitfähigem Siliziumnitrid gefertigt ist und einen Elektrodenabschnitt 20 bildet, auf, der ein Lumen 24 und eine endseitige Düse 25 aufweist. Damit kann ein Gerät für die Flüssigkeitsstrahlchirurgie (Wasserstrahlchirurgie) hergestellt werden. Am Ende des Elektrodenabschnittes 20 ist unter Zuhilfenahme des zuvor geschilderten Verfahrens ein halbkugelförmiger Trägerabschnitt 10 (Fig. 7) bzw. ein Haken 11 (Fig. 8 und 9) ausgebildet, die jeweils zum Präparieren von Gewebe dienen können. Die in Fig. 7 bis 9 gezeigten Instrumente weisen also drei Benutzungsmöglichkeiten auf. Zum einen kann mit dem Instrument mechanisch präpariert werden, zum zweiten kann mittels eines Wasserstrahls Gewebe getrennt werden und zum dritten kann mit Hilfe von hochfrequenten Strömen Gewebe geschnitten und koaguliert werden.

Bei der in Fig. 10 gezeigten Ausführungsform der Erfindung handelt es sich um den Endabschnitt (im Längsschnitt) einer Fasszange. Hier sind die zwei Schenkel der Fasszange als Trägerkörper 10 ausgebildet und nur an den Endabschnitten sind Elektrodenabschnitte 20 aus leitender Siliziumnitrid-Keramik auf die nichtleitende Siliziumnitrid-Keramik aufgebracht. Die Kontaktierung erfolgt hierbei durch Leitungen 26, welche während der Herstellung des Verbundgrünlings mit umspritzt werden.

Die Flächen der elektrisch leitenden Elektrodenabschnitte 20 können poliert oder aber geriffelt sein, wobei ein derartiger Verfahrensschritt einer Oberflächenbearbeitung vorzugsweise am Verbundgrünling durchgeführt wird.

### Bezugszeichenliste

- 10: Trägerabschnitt
- 11: Haken
- 20: Elektrodenabschnitt
- 21: Metallisierung
- 22: Öffnung
- 23: Hinterschneidung
- 24: Lumen
- 25: Düse
- 26: Leitung

## Patentansprüche

1. Elektrodeneinrichtung eines elektrochirurgischen Instruments, umfassend mindestens einen leitfähigen Elektrodenabschnitt (20) und einen elektrisch isolierenden Trägerabschnitt (10), wobei der Elektrodenabschnitt (20) ebenso wie der Trägerabschnitt (10) aus einem keramischen Material gefertigt sind, und wobei ein Grünling des Trägerabschnitts (10) zusammen mit einem Grünling des Elektrodenabschnitts (20) miteinander zu einem einzigen Verbundgrünling verbunden und gemeinsam gesintert sind, **dadurch gekennzeichnet, dass** der Elektrodenabschnitt (20) und der Trägerabschnitt (10) aus Keramikmaterial mit im Wesentlichen denselben Wärmeausdehnungskoeffizienten, insbesondere aus Siliziumnitrid, aber mit unterschiedlichen Dotierungen oder Beimischungen Leitfähigkeit erzeugender Bestandteile gefertigt sind.

2. Elektrodeneinrichtung Anspruch 1, **dadurch gekennzeichnet, dass** der Grünling des Trägerabschnitts (10) zusammen mit dem Grünling des Elektrodenabschnitts (20) miteinander stofflich zu einem einzigen Verbundgrünling verbunden und gemeinsam gesintert sind

3. Elektrodeneinrichtung nach einem der vorhergehenden Ansprüche, **dadurch gekennzeichnet, dass** das keramische Material des Elektrodenabschnittes Beimischungen/Dotierungen von Aluminiumoxid und/oder Yttriumoxid und/oder Magnesiumoxid aufweist.

4. Elektrodeneinrichtung nach einem der vorhergehenden Ansprüche, **dadurch gekennzeichnet, dass** der Verbundgrünling vor dem Sintern einer Oberflächenbehandlung, insbesondere Lasern oder einer spanabhebenden Verformung, z. B. Gleitschleifen unterworfen ist.

5. Elektrodeneinrichtung nach einem der vorhergehenden Ansprüche, **dadurch gekennzeichnet, dass** der Elektrodenabschnitt (20) mit dem Trägerabschnitt (10) unter Bildung von Hinterschneidungen (22 , 23) zur Stabilitätssteigerung verzahnt ist.

6. Elektrodeneinrichtung nach einem der vorhergehenden Ansprüche, **dadurch gekennzeichnet, dass** der Elektrodenabschnitt (20) mindestens teilweise beschichtet, insbesondere metallisiert, insbesondere galvanisiert ist.

7. Verfahren zur Herstellung einer Elektrodeneinrichtung eines elektrochirurgischen Instrumentes, umfassend die Schritte
a. Zurverfügungstellen eines elektrisch isolierenden ersten Keramikmaterials;
b. Zurverfügungstellung eines elektrisch leitenden zweiten Keramikmaterials;
c. Herstellen eines Verbundgrünlings, der mindestens einen Trägerabschnitt aus dem ersten Keramikmaterial und mindestens einen Elektrodenabschnitt aus dem zweiten Keramikmaterial umfasst;
d. gemeinsames Sintern des ersten und zweiten Keramikmaterials, die den Verbundgrünling bilden;
**dadurch gekennzeichnet, dass** das erste und das zweite Keramikmaterial im Wesentlichen dieselben Wärmeausdehnungskoeffizienten aufweisen und dasselbe keramische Material aber mit unterschiedlichen Dotierungen oder Beimischungen von Leitfähigkeit erzeugenden Bestandteilen umfassen.

8. Verfahren nach Anspruch 7, **dadurch gekennzeichnet, dass** das keramische Material Siliziumnitrid umfasst,

9. Verfahren nach Anspruch 7 oder 8, **dadurch gekennzeichnet, dass** der Verbundgrünling vor dem Sintern einer Oberflächenbehandlung, insbesondere Lasern oder einer spanabhebenden Verformung, z. B. Gleitschleifen unterworfen wird.

10. Verfahren nach einem der Ansprüche 7 bis 9, **dadurch gekennzeichnet, dass** der Grünling in einem 2K-Spritzgussverfahren hergestellt wird.

## Claims

1. Electrode arrangement of an electrosurgical instrument, comprising at least one conductive electrode portion (20) and an electrically isolating carrier portion (10), wherein the electrode portion (20) and the carrier portion (10) are made from a ceramic material, and wherein a green body of the carrier portion (10) together with a green body of the electrode portion (20) are connected together to form a single composite green body and are sintered jointly, **characterised in that** the electrode portion (20) and the carrier portion (10) are made from ceramic material with substantially the same thermal expansion coefficient, in particular from silicon nitride, but with different dopings or admixtures of constituents generating conductivity.

2. Electrode arrangement according to claim 1, **characterised in that** the green body of the carrier portion (10) together with the green body of the electrode portion (20) are connected together materially into a single composite green body and are sintered jointly.

3. Electrode arrangement according to one of the preceding claims, **characterised in that** the ceramic material of the electrode portion comprises admixtures/dopings of aluminium oxide and/or yttrium oxide and/or magnesium oxide.

4. Electrode arrangement according to any of the preceding claims, **characterised in that** before sintering, the composite green body is subjected to a surface treatment, in particular laser treatment, or a material-removal deformation, e.g. vibratory grinding.

5. Electrode arrangement according to any of the preceding claims, **characterised in that** the electrode portion (20) is intermeshed with the carrier portion (20), forming undercuts (22, 23) to increase the stability.

6. Electrode arrangement according to any of the preceding claims, **characterised in that** the electrode portion (20) is at least partially coated, in particular metallised, in particular galvanised.

7. Method for producing an electrode arrangement of an electrosurgical instrument, comprising the steps:
a) provision of an electrically isolating first ceramic material;
b) provision of an electrically conductive second ceramic material;
c) production of a composite green body comprising at least one carrier portion of the first ceramic material and at least one electrode portion of the second ceramic material;
d) joint sintering of the first and second ceramic materials which form the composite green body;
**characterised in that** the first and the second ceramic materials have substantially the same thermal expansion coefficient and comprise the same ceramic material but with different dopings or admixtures of constituents generating conductivity.

8. Method according to claim 7, **characterised in that** the ceramic material comprises silicon nitride.

9. Method according to claim 7 or 8, **characterised in that** before sintering, the composite green body is subjected to a surface treatment, in particular laser treatment, or a material-removal deformation, e.g. vibratory grinding.

10. Method according to any of claims 7 to 9, **characterised in that** the green body is produced in a 2K injection moulding process.

## Revendications

1. Dispositif à électrodes d'un instrument d'électrochirurgie, comprenant au moins une partie d'électrode (20) conductrice et une partie de support (10) électriquement isolante, sachant que la partie d'électrode (20), de même que la partie de support (10), est constituée d'un matériau céramique, et sachant qu'une ébauche crue de la partie de support (10) et une ébauche crue de la partie d'électrode (20) sont reliées l'une à l'autre pour former une ébauche crue composée unique et sont frittées ensemble, **caractérisé en ce que** la partie d'électrode (20) et la partie de support (10) sont fabriquées à partir de matériaux céramiques présentant sensiblement les mêmes coefficients de dilatation thermique, notamment à partir de nitrure de silicium, mais avec des dopages ou des ajouts différents de constituants générant de la conductivité.

2. Dispositif à électrodes selon la revendication 1, **caractérisé en ce que** l'ébauche crue de la partie de support (10) et l'ébauche crue de la partie d'électrode (20) sont liées l'une à l'autre par matière, pour former une ébauche crue composée unique, et sont frittées ensemble.

3. Dispositif à électrodes selon l'une des revendications précédentes, **caractérisé en ce que** le matériau céramique de la partie d'électrode présente des ajouts/dopages d'oxyde d'aluminium et/ou d'oxyde d'yttrium et/ou d'oxyde de magnésium.

4. Dispositif à électrodes selon l'une des revendications précédentes, **caractérisé en ce que**, avant le frittage, l'ébauche crue composée est soumise à un traitement de surface, en particulier à un traitement au laser ou à une mise en forme par enlèvement de matière, par exemple par polissage mécano-chimique.

5. Dispositif à électrodes selon l'une des revendications précédentes, **caractérisé en ce que** la partie d'électrode (20) engrène avec la partie de support (10) en formant des contre-dépouilles (22, 23), afin d'augmenter la stabilité.

6. Dispositif à électrodes selon l'une des revendications précédentes, **caractérisé en ce que** la partie d'électrode (20) est pourvue au moins en partie d'un revêtement, en particulier par métallisation, notamment par galvanisation.

7. Procédé de fabrication d'un dispositif à électrodes d'un instrument d'électrochirurgie, comprenant les étapes suivantes :
a. mise à disposition d'un premier matériau céramique électriquement isolant ;
b. mise à disposition d'un deuxième matériau céramique électriquement conducteur ;
c. réalisation d'une ébauche crue composée qui comprend au moins une partie de support, constituée du premier matériau céramique, et au moins une partie d'électrode constituée du deuxième matériau céramique ;
d. frittage commun du premier et du deuxième matériau céramique qui forment l'ébauche crue composée ;
**caractérisé en ce que** les premier et deuxième matériaux céramiques présentent sensiblement les mêmes coefficients de dilatation thermique et comportent le même matériau céramique, mais avec des dopages ou des ajouts différents de constituants générant de la conductivité.

8. Procédé selon la revendication 7, **caractérisé en ce que** le matériau céramique comprend du nitrure de silicium.

9. Procédé selon la revendication 7 ou 8, **caractérisé en ce que**, avant le frittage, l'ébauche crue composée est soumise à un traitement de surface, en particulier à un traitement au laser ou à une mise en forme par enlèvement de copeaux, par exemple par polissage mécano-chimique.

10. Procédé selon l'une des revendications 7 à 9, **caractérisé en ce que** l'ébauche crue est fabriquée au cours d'un procédé de moulage par injection 2K.
